# EUROPEAN PATENT SPECIFICATION

(11) **EP 0 494 042 B1**
(45) Date of publication and mention of the grant of the patent: **18.06.1997**
(21) Application number: 91630120.3
(22) Date of filing: 30.12.1991
(51) Int. Cl.: A61M 5/155

(54) **Liquid delivery device particularly useful for delivering drugs**
Flüssigkeitsabgabevorrichtung für Medikamentenverabreichung
Appareil de distribution de liquides pour administration de médicaments

(30) Priority: 31.12.1990 IL 96835; 06.03.1991 IL 97457; 21.08.1991 IL 99262
(43) Date of publication of application: 08.07.1992
(73) Proprietor: S.I. SCIENTIFIC INNOVATIONS Ltd., 49 511 Petah Tikva (IL)
(72) Inventor: Gross, Joseph, I-73 160 Moshav Mazor (IL); Zucker, Shlomo, I-40297 Mihmoret (IL)
(74) Representative: Schmitz, Jean-Marie

(56) References cited:
- EP-A- 0 112 585
- EP-A- 0 385 916
- FR-A- 2 195 461
- US-A- 4 902 278

## Description

The present invention relates to liquid delivery devices, and particularly to devices for delivering liquids containing drugs. The drugs may be in solution and/or suspension in a pharmaceutically acceptable medium which is in the liquid state under normal temperature and pressure.

In US-A-5,062,834, there is disclosed a liquid delivery device according to the preamble of claim 1. More specifically, this prior US Patent 5,062,834 discloses a liquid delivery device including an outer housing having an outlet for the liquid to be delivered, a displaceable member within the housing defining a first contractible chamber on one side of the displaceable member for holding a supply of the material to be delivered via the outlet, and a second contractible chamber on the opposite side of the displaceable member. The device further includes pressure-control means for controlling the pressure produced in the second contractible chamber for controlling the displacement of the displaceable member, and thereby the rate of flow of the liquid via the outlet. In the device described in that patent application, the pressure-control means comprised an electrolytic cell having a pair of electrodes separated by an electrolyte capable of generating a gas applied to the second chamber according to the electrical current passing through the electrolyte.

A drawback in the device described in that patent is that the rate of flow of the liquid (e.g., a drug) via the outlet is very sensitive to variations in ambient temperature, and/or to any leakage in the pressure control chamber.

Compensation for changes in ambient temperature and pressure are known in pulsatile pumps, such as described in EP-A-0 112 585, e.g. by measuring absolute pressure in order to adjust the pulsing rate of the pulsatile pump. However, the liquid delivery device described in the abovementioned US-A-5,062,834 does not deliver the liquid in a pulsatile manner, but rather in a continuous manner at a rate dependent on the pressure in the second contractible chamber, and therefore the manner of compensating for changes in ambient temperature and pressure used in pulsatile pumps cannot be applied to this type of liquid delivery device.

An object of the present invention is to provide a liquid delivery device of the type described in US-A-5,062,834 which is less sensitive to variations in ambient temperature.

According to the present invention, there is provided a liquid delivery device as described above, characterized in that the housing further includes: a second displaceable member defining a third contractible chamber between the housing and the first contractible chamber adjacent to the outlet; and a control valve having two valve members normally spaced apart for controlling the flow to the outlet; one of the valve members being on the second displaceable member such that changes in ambient temperature produced in the first and third chambers produce substantially equal and opposite forces on the opposite sides of the displaceable member whereby the control valve compensates the rate of flow to the outlet for changes in ambient temperature.

The invention is herein described, by way of example only, with reference to the accompanying drawings, wherein:
Fig. 1 is a longitudinal sectional view illustrating one form of drug delivery device constructed in accordance with the present invention, Fig. 1a being an enlarged fragmentary view of a portion of the device of Fig. 1;
Figs. 2, 3, 4 and 5 are longitudinal sectional views illustrating four further forms of drug delivery devices constructed in accordance with the present invention;
Figs. 4a and 4b are top and perspective views, respectively, of the calibrating disc used in the device of Fig. 4;
Fig. 5a is an exploded view of the device of Fig. 5;
Fig. 6 is a top plan view illustrating any one of the devices of Figs. 1-5 adapted to be worn on a wrist and including a control circuit for controlling the delivery of the drug; and
Fig. 7 is a block diagram illustrating the control circuit included in the device of Fig. 6.

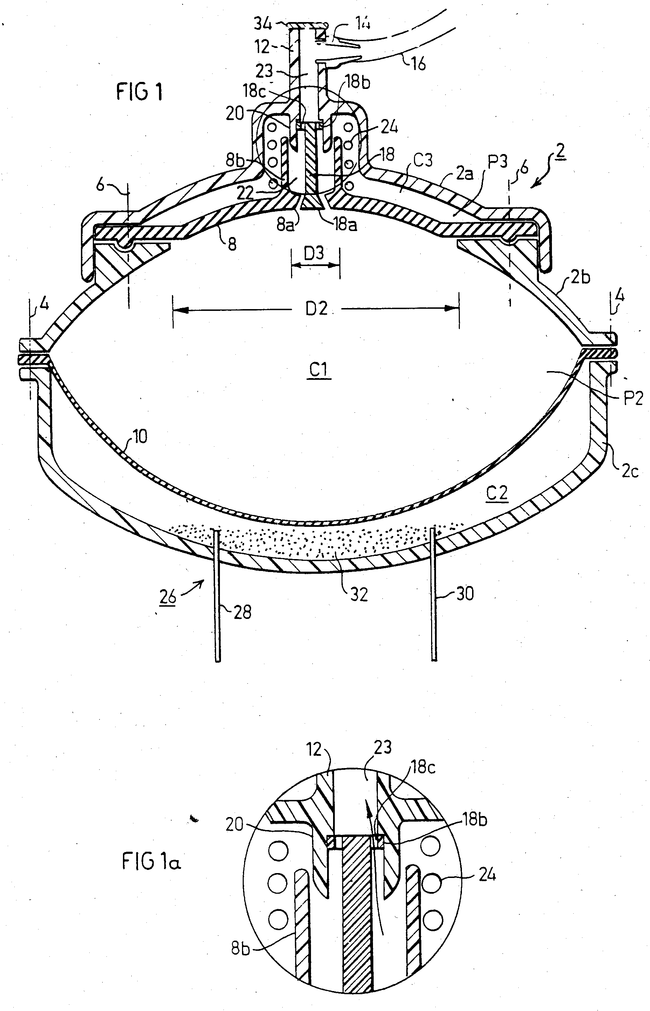

The drug delivery device illustrated in Fig. 1 includes an outer rigid housing, generally designated 2, constituted of three sections 2a, 2b, 2c, all secured together in any suitable manner, e.g., by fasteners 4 and 6. The three housing sections 2a, 2b, 2c are preferably of circular cross-section. A first diaphragm 8 is clamped between housing sections 2a and 2b, and a second diaphragm 10 is clamped between housing sections 2b and 2c.

It will be seen that diaphragm 10 divides the interior of housing 2 into a first contractible chamber C₁ on one side of the diaphragm, and a second contractible chamber C₂ on the opposite side of the diaphragm; and that diaphragm 8 defines a third contractible chamber C₃ between it and housing section 2a. As will be described more particularly below, chamber C₁ serves to hold a supply of the liquid (e.g., containing a drug) to be delivered; chamber C₂ serves as a pressure-control chamber for controlling the rate of delivery of the drug; and chamber C₃ serves as a compensating chamber for reducing the sensitivity of the delivery rate to variations in ambient pressure and temperature.

Housing section 2a is formed with a tubular extension 12 having an outlet opening 14 adapted to receive a flexible tube 16 for delivering the drug to a subject. A stem 18 is fixed to the tubular extension 12 and extends inwardly into chamber C₁ through an opening 8a in diaphragm 8. The inner tip of stem 18 is formed with a conical head 18a, and the opening 8a through diaphragm 8 is of a complementary conical configuration, such that this opening, together with conical head 18a of stem 18, serves as a control valve between chamber C₁ and the outlet tube 16 connected to the subject.

Tubular extension 12 includes an inwardly-extending section 20 to which the inner end 18b of stem 18 is fixed. Diaphragm 8 is further formed with a tubular section 8b enclosing section 20 to define a passageway 22 for the liquid leaving chamber C₁. The fixed end 18b of stem 18 is formed with openings, as shown at 18c in Fig. 1a, to permit the liquid to flow through passageway 23 within the tubular extension 12, and from there to the outlet 14. The valve formed by stem head 18a and diaphragm opening 8a is biassed to its closed condition by a coiled spring 24 within chamber C₃ and interposed between diaphragm 8 and housing section 2a adjacent its tubular extension 12.

As indicated earlier, chamber C₂ between diaphragm 10 and housing section 2c serves as a pressure-control chamber for controlling the displacement of diaphragm 10, and thereby the rate of flow of the liquid from chamber C₁ to the outlet 14. For this purpose, chamber C₂ includes an electrolytic cell, generally designated 26, having a pair of electrodes 28, 30 separated by an electrolyte 32 capable of generating a gas within chamber C₂ according to the electrical current passing through the electrolyte. The electrolyte 32 is disposed within chamber C₂, whereas the electrodes 28, 30 pass outwardly through housing section 2c to enable them to be connected to an external electric supply, e.g., a battery.

The end of the tubular extension 12 formed with the outlet 14 is closed by a semi-permeable cap 34 which is permeable to gas, but not to liquids. Cap 34 serves to vent to the atmosphere any gas in chamber C₁, and thereby prevents gas from being included in the drug delivered via outlet 14 to the subject.

The drug delivery device illustrated in Figs. 1 and 1a operates as follows:

Chamber C₁ is first completely filled with the liquid containing the drug to be dispensed. When the drug is to be delivered, electrodes 28, 30 are connected to a power supply (e.g., a battery), and electric current is applied thereby to the electrolyte 32 within chamber C₂, which electrolyte generates a gas according to the magnitude of the electric current applied. The gas generated in chamber C₂ displaces diaphragm 10 inwardly, thereby forcing out liquid from chamber C₁, via valve opening 8a in diaphragm 8, at a rate corresponding to the rate of gas generation in chamber C₂. The dispensed liquid flows via passageways 22 and 23 to the outlet tube 16.

As indicated earlier, the rate of delivery of the liquid from chamber C₁ is affected, not only by the rate of gas generation within chamber C₂, but also by the ambient temperature and pressure prevailing at the time the device is operated. The variations of temperature and pressure are compensated for in the device illustrated in Fig. 1 in the following way:

The variations in temperature substantially affect both sides of diaphragm 8 in the same manner; thus, an increase in temperature with respect to chamber C₁, tending to displace diaphragm 8 in the direction of increasing the size of the valve opening 8a, will also be applied to the opposite side of diaphragm 8 tending to decrease the size of the valve opening, thereby substantially cancelling the effects of temperature variations.

The variations in pressure will affect only the portion (D₃) of diaphragm 8 exposed to the atmospheric pressure via the outlet 14. However, since the complete diaphragm diameter (D₂) is much greater than portion D₃, the variations in pressure will thus have little affect in changing the flow rate via the valve opening 8a.

The semi-permeable cap 34 at the end of the extension tube 12 vents gas to the atmosphere but not liquid. Accordingly, should gas enter chamber C₁ (e.g., because of a leakage, or a rupture of diaphragm 10), such gas will be vented via cap 34 and therefore will not be included in the drug delivered to the subject via outlet 14. Cap 34 may also be used for initially priming the pump.

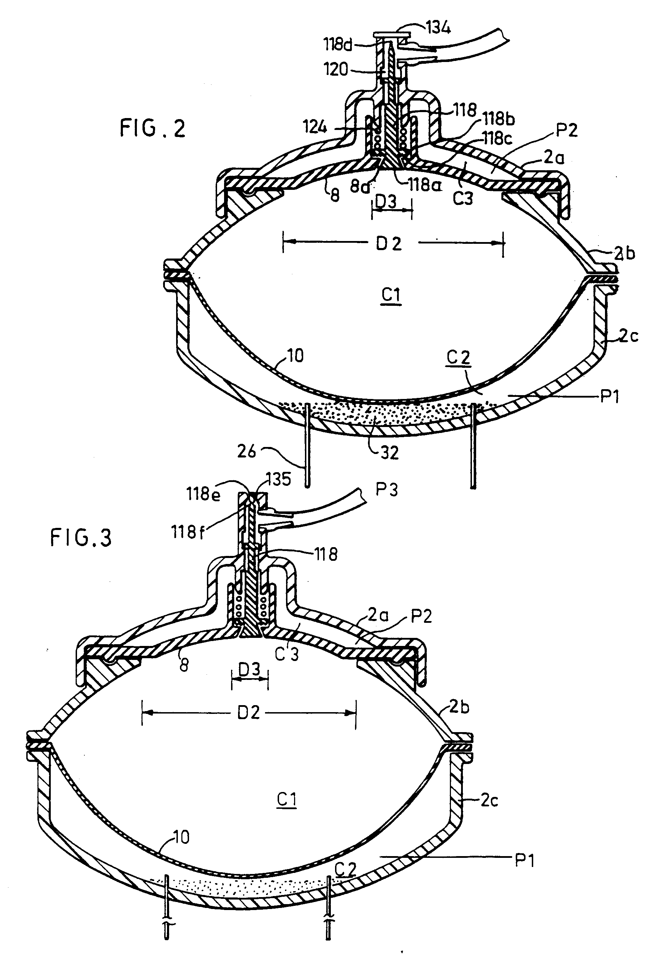

The drug delivery device illustrated in Fig. 2 is constructed and operates in a substantially similar manner as described above with respect to Fig. 1; to facilitate understanding, its parts which are generally the same as those in Fig. 1 are identified by the same reference numerals.

In the drug deliver device illustrated in Fig. 2, the stem, identified as 118, includes a conical head 118a serving as the valve member cooperable with valve opening 8a in diaphragm 8, in the same manner as described above with respect to Fig. 1. Stem 118, however, is integrally formed with an annular ring 118b serving as a shoulder for the spring 124, which spring biasses ring 118b towards diaphragm 8. The underface of ring 118b is formed with ribs 118c, to permit liquid from chamber C₁ to flow into passageway 120 to the outlet 114.

The opposite end 118d of stem 118 is pointed and is normally spaced from the venting cap 134. In this case, the venting cap 134 is of a material which is pierceable by the stem pointed end 118d upon the excessive displacement of the stem.

Thus, spring 124 and diaphragm 8 will reduce the sensitivity of the delivery device to variations in temperature and pressure in the same manner as described above with respect to Fig. 1.

In addition, should the supply of the drug within chamber C₁ becomes exhausted, or should an excessive pressure be otherwise produced in chamber C₁ (e.g., by an occlusion downstream of the outlet 114), stem 118 will be displaced so that its pointed end 118d pierces cap 134, thereby causing the drug within the passageway 120 to flow out through the vent, rather than via the outlet 114.

Fig. 3 illustrates a constuction very similar to that of Fig. 2, and therefore its parts are identified by the same reference numerals. In the construction of Fig. 3, however, cap 134 is replaced by a second valve including a valve opening, identified as 135, formed in the extension 12 (instead of in cap 134), cooperable with the end of valve stem 118. Valve stem 118 is formed with a tip 118e which, during the normal operation of the device, is located in valve opening 135 to close the vent, but upon the excessive displacement of stem 118, positions an annular recess 118f within opening 135, thereby opening the vent.

In all other respects, the drug delivery device illustrated in Fig. 3 is constructed and operates in the same manner as described above.

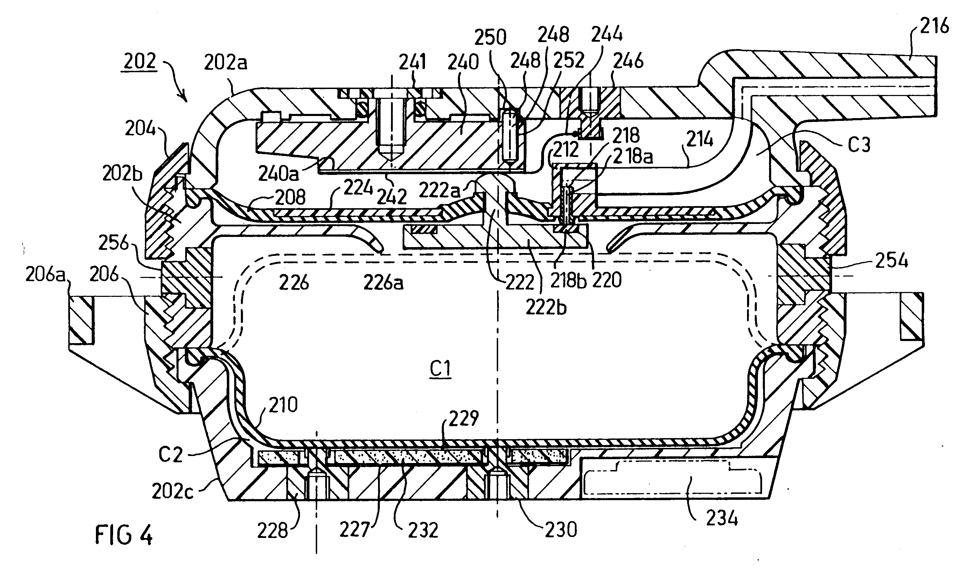

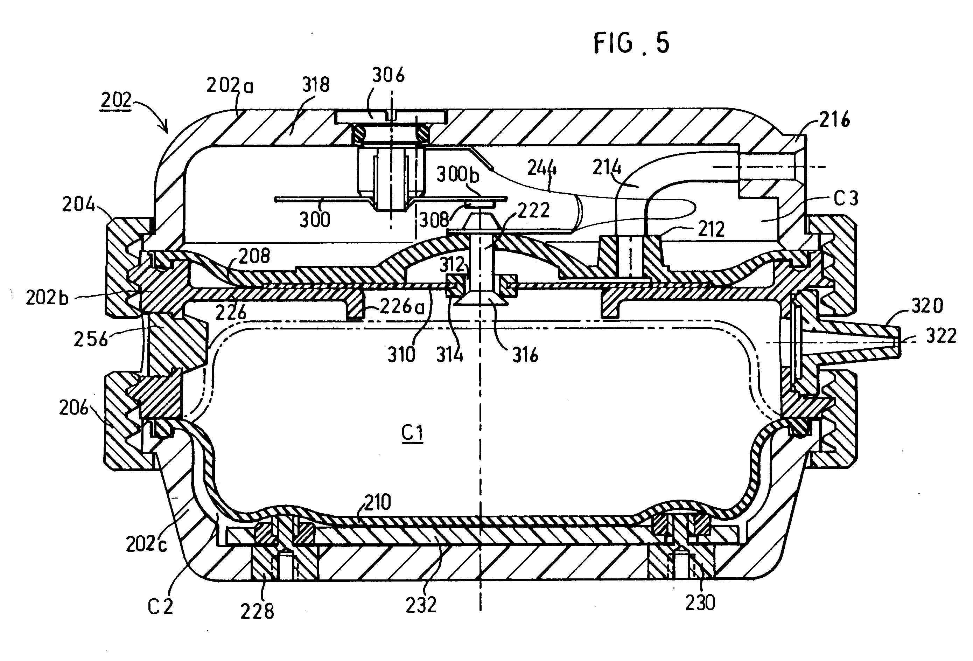

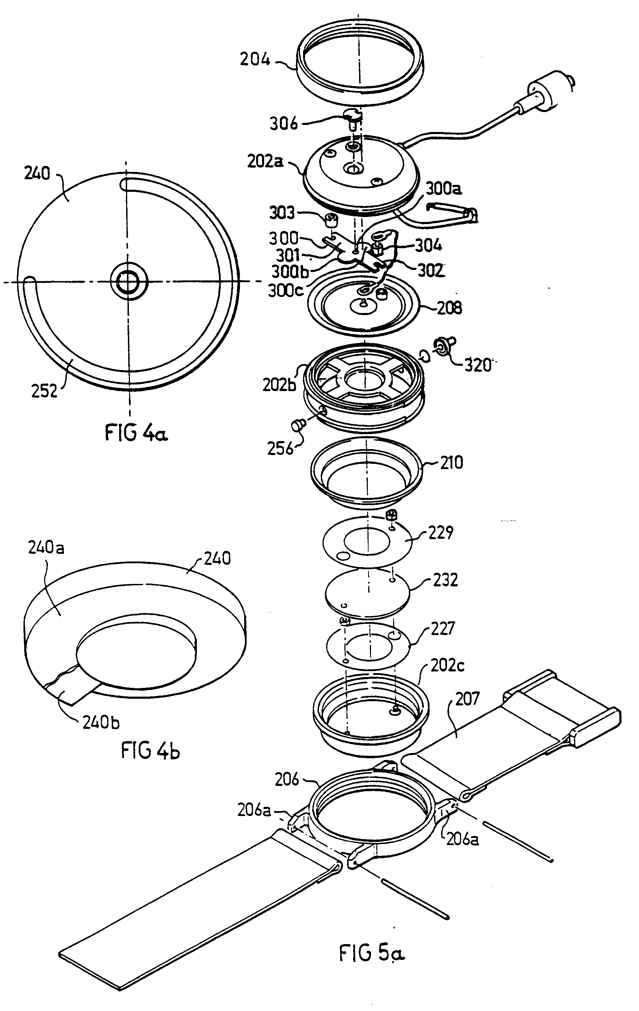

Fig. 4 illustrates another form of drug delivery device constructed in accordance with the present invention but provides a number of additional features as compared to the devices of Figs. 1-3.

Thus, the device illustrated in Fig. 4 also includes an outer rigid housing 202 constituted of three sections 202a, 202b, 202c secured together by a pair of bevel rings 204, 206. Bezel ring 206 is provided with extensions 206a at its opposite ends to receive a wrist band and thereby to permit the device to be worn on the wrist of the user. A diaphragm 208 is clamped between housing sections 202a and 202b, and a second diaphragm 210 is clamped between housing sections 202b and 202c. The two diaphragms 210 and 208 divide the interior of housing 202 into a liquid (e.g., drug) containing chamber C₁, a pressure-control chamber C₂ and a compensating chamber C₃, as in the previously-described embodiments.

Diaphragm 208 carries a fitting 212 connected by a flexible tube 214 to an outlet opening 216 through which the drug is delivered to a subject. Fitting 212 further includes a valve member 218 formed with an axial bore 218a and a conical head 218b engageable with a valve seat 220. The valve seat 220 is of annular configuration fixed to a metal stem 222 carried by the central portion of diaphragm 208. Stem 222 is formed with an enlarged head 222a disposed within the compensating chamber C₃, and with a disc section 222b located in the drug chamber C₁.

Seat 220 is carried on the upper surface of disc section 222b of stem 222 in alignment with valve member 218. Fitting 212 carrying the valve member is integrally formed with a rigid annular member 224 fixed to the upper surface of diaphragm 208, and serves to stiffen the diaphragm. Housing section 202b is integrally formed with an annular member 226 underlying diaphragm 208 to limit the displacement of the diaphragm. The central part of annular member 226 is turned inwardly into compartment C₁ below the outer face of disc 222b of the stem 222, to prevent the lower diaphragm 210 from engaging the underface of the disc.

Chamber C₂, between diaphragm 210 and housing section 202c, contains an electrolytic cell having a pair of electrodes 227, 229 having terminals 228, 230 and separated by an electrolyte 232 capable of generating a gas within chamber C₂ according to the electrical current passing through the electrolyte. Housing section 202c, through which the two electrodes 228, 230 pass, may also be formed with a compartment 234 for receiving one or more batteries to energize the electrolytic cell.

A calibrating member 240 is carried by housing section 202a and cooperates with stem 222 carried by diaphragm 208. Calibrating member 240 is in the form of a disc which may be rotated by a screw 241. The lower surface of calibrating disc 240 is cut with a helical rib 240a of increasing thickness (Fig. 4b) engaged by the head 222a of stem 222, such that rotating the calibrating disc 240 by screw 241 will displace the stem with respect to diaphragm 208 more or less into the drug chamber C₁.

Calibrating screw 241 and disc 240 are preferably made of metal so as to provide electrical continuity to the underface of the disc. A layer of a pressure-sensitive resistor material 242, such as a silicone rubber-graphite composition, is carried by the helical rib 240a of the calibrating disc 240. The calibrating screw 241 serves as one terminal of an electrical circuit which includes the metal calibrating disc 240 and the layer 240b of pressure-sensitive resistor material. Layer 240b is connected by an electrical conductor 244 to another electrical terminal 246 of the electrical circuit.

The rotation of calibrating disc 240 is guided by a pin 248 having one end received within a recess 250 formed in housing section 202a, and the opposite end received within an annular groove 252 (Fig. 4a) formed partially around the circumference of the calibrating disc.

Housing section 202b further includes a drug-injection port 256 for injecting a drug into chamber C₁, and vent 254, such as a hydrophobic filter, which is permeable to air but not to liquid.

The device illustrated in Fig. 4 is used in the following manner:

The device is first precalibrated for a particular temperature and pressure. This may be done by applying a predetermined air pressure via the hydrophobic filter 256, and rotating the calibrating screw 241 until diaphragm 208 is displaced to open valve 218. As one example, the device may be precalibrated for 400 mm water (e.g., 0.04 atmospheres) in this manner.

The liquid drug to be delivered is then introduced into chamber C₁ by injection via port 256. During this introduction of the drug, the air within chamber C₁ is vented to the atmosphere via the hydrophobic filter 254. As soon as the pressure within chamber C₁ reaches the precalibrated pressure, valve 218 opens, so that liquid exits through the outlet 216. This provides an indication that the device has been primed.

In use, electrodes 228, 230 are connected to batteries 234 and cause the electrolyte 232 to generate a gas in chamber C₂ corresponding to the current supplied. In the event there was some pressure loss from chamber C₁ (e.g., because of a leakage), head 222a of the stem 222 will not be in engagement with the pressure-sensitive resistor layer 242 carried by the underside of the calibrating disc 240, and this fact will be indicated by the electrical signal outputted from the two terminals 241, 246 connected together via the pressure-sensitive layer 242 and electrical lead 244. Also, valve 218 will be closed on the valve seat 220, so that no liquid will be dispensed from chamber C₁.

Diaphragm 208 forms a yielding connection between the valve seat 220 and the valve member 218 such that, as soon as diaphragm 208 moves the stem head 222a to contact the pressure-sensitive resistor layer 242 of the calibrating disc 240, valve member 218 opens with respect to valve seat 220 to start the delivery of liquid from chamber C₁. The pressure applied by stem head 222a against the pressure-sensitive resistor layer 242 reduces the resistance of that layer. An electrical signal is thus outputted between the two terminals 241, 246, indicating the start of delivery of the drug from compartment C₁ through the outlet 216.

In case of an overpressure occurring in the drug chamber C₁, such as may be caused by an occlusion in the feeding of the drug to the patient or by the exhaustion of the drug from chamber C₁, this will be sensed by the pressure applied by stem head 222a against the pressure-sensitive resistor layer 242. In such a case, the device could be automatically turned-off, and/or an alarm or indicator could be energized. In addition, by providing the operating pressure of the control valve always higher than the head pressure of the supply tube, the influence of the supply tube position is eliminated relative to the pump.

It will thus be seen that the amount of drug delivered via outlet 216 can be easily controlled by the electrical current applied to the two electrodes 228, 230 of the electrolytic cell including the electrolyte 232. It will also be seen that the described arrangement, particularly the stem 222 and the valve 218 both carried by the diaphragm 208, not only compensates for variations in the ambient temperature in the same manner as described above with respect to Fig. 1, but also senses the actual start of delivery of the drug so that precise quantities can be delivered at precise rates.

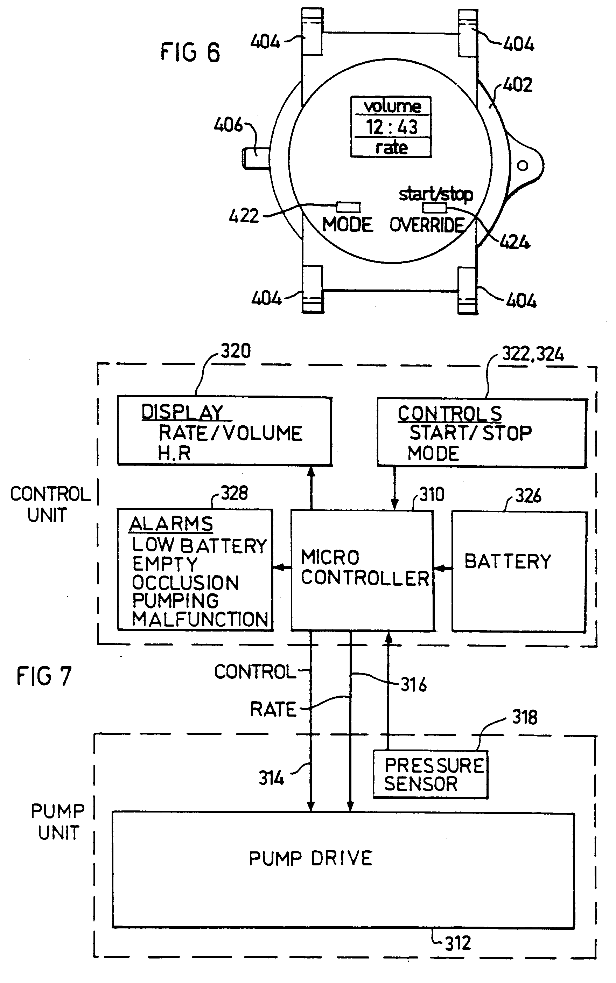

The device illustrated in Figs. 5 and 5a is similar to that of Fig. 4, and those parts which are generally similar to those in Fig. 4 are correspondingly numbered. A main difference in the construction of the device of Figs. 5 and 5a is in the calibrating mechanism.

Thus, instead of using a calibrating disc (240, Fig. 4), the calibrating mechanism in the device of Figs. 5 and 5a includes a leaf spring 300 which is fixed at its opposite ends 301, 302 to the upper housing section 202a by a pair of fasteners 303, 304. A pin 306 is threaded through the upper housing section 202a and through the center 300a of the leaf spring, such that the leaf spring is bowed at its center, and its center may be moved towards and away from the upper diaphragm 208 by threading pin 306 more or less into the housing section 202a. The bowed central, section of leaf spring 300 is formed with a pair of laterally-extending tabs 300b, 300c, one of which overlies the stem 222 received in the upper diaphragm 208. The tab (e.g., 300b) overlying stem 222 is provided with a layer of a pressure-sensitive resistor 308, of the same material and for the same purpose as the pressure-sensitive resistor layer 242 in Fig. 4.

Another difference in the device of Figs. 5 and 5a, as compared to that of Fig. 4, is that a rigid reinforcing disc 310 is bonded to the undersurface of the upper diaphragm 208. The center of the rigid disc 310 is formed with an opening 312 lined with a rubber ring 314 serving as a valve seat, and stem 222 extends through opening 312 and terminates in a conical head 316 cooperable with valve seat 314.

It will thus be seen that in the device of Figs. 5 and 5, threaded pin 306, threadedly engaging the center of the bowed leaf spring 300, serves as the calibrating means, corresponding to calibrating disc 240 in Fig. 4; and that the conical head 316 of stem 222, cooperable with valve seat 314, serves as the auxiliary valve corresponding to valve member 218 and valve seat 220 in Fig. 4. In addition, the electrical conductor 244 or sensing contact of the stem 222 with the calibrating leaf spring 300, particularly the pressure-sensitive resistor 308 on tab 300b of the spring, connects the calibrating screw 306 to the metal stem 222, and when the stem contacts the pressure-sensitive resistor 308, the ciruit is completed to the leaf spring 300 connected externally by a connector shown schematically at 318 in Fig. 5.

Another minor difference in the Figs. 5, 5a device over that of Fig. 4 is that the vent in chamber C₁ is in the form of a nipple 320 closed by a hydrophobic filter 322.

The device illustrated in Figs. 5 and 5a is otherwise constructed and operates in substantially the same manner as the device of Fig. 4. As shown particularly in Fig. 5a, the bevel ring 206 may also be provided with extensions 206a on its opposite sides for receiving a band to enable wearing the device on the user's wrist.

Figs. 6 and 7 illustrate an embodiment of the invention wherein the drug delivery device is equipped wih an electronic control circuit which permits the drug to be delivered to the user at preprogrammed times and rates.

As shown in Fig. 6, the device includes a housing 402 also provided with extensions 404 for receiving a wrist band to enable the device to be worn on the user's wrist. The interior of housing may be constructed as described above with respect to any one of Figs. 1-5. The housing 402 is provided with a drug delivery outlet 406, and also with a port (not shown) which is permeable to gas but not to liquid.

The upper part of the housing includes the electronic control circuit for controlling the time and rate of drug delivery via the outlet 406. As shown in Fig. 7, the control circuit includes a microcontroller 310 which controls the pump drive, namely the electrical current supplied to the two electrodes of the electrolytic cell to cause the electrolyte to generate the propellant gas. The start and stop signals are fed by the microcontroller 310 to the pump drive (electrolytic cell electrodes) via conductor 314, and a signal indicating the magnitude of the electrical current supplied, which determines the rate of drug delivery, is fed to the pump drive via conductor 316. The actual start of drug delivery is sensed by the pressure sensor 318 (e.g., stem head 222a engaging the pressure-sensitive layer 242 in the Fig. 4 embodiment), so that the microcontroller can keep an accurate measurement of the actual quantity of drug delivered. The volume and rate of drug delivery is displayed in a display 320 viewable on the face of the device as shown in Fig. 6. The face of the device further includes a mode switch 422 which permits preprogramming the quantities and times of drug delivery, and an override start/stop switch 424 to permit manual control. The device further includes a battery supply 326, an alarm 328 (e.g., visual and/or audible) to indicate various conditions such as low battery, empty, occlusion, pumping, and malfunction.

## Claims

1. A liquid delivery device including: an outer housing (2; 202) having an outlet (14; 214) for the liquid to be delivered; a first displaceable member (10; 210) within the housing defining a first contractible chamber (C₁) on one side of the first displaceable member for holding a supply of the liquid to be delivered, and a second contractible chamber (C₂) on the opposite side of the first displaceable member; and pressure control means (26) for controlling the pressure produced in said second contractible chamber for controlling the displacement of the first displaceable member, and thereby the rate of flow of the liquid via said outlet; characterized in that said housing further includes: a second displaceable member (8; 208) defining a third contractible chamber (C₃) between said housing and said first contractible chamber adjacent to said outlet; and a control valve having two valve members (8a, 18; 8a, 118a; 218a, 220; 314, 316) normally spaced apart for controlling the flow to said outlet; one of said valve members (8a, 218a, 314) being on said second displaceable member such that changes in ambient temperature produced in the first and third chambers produce substantially equal and opposite forces on the opposite sides of the displaceable member whereby the control valve compensates the rate of flow to said outlet for changes in ambient temperature.

2. The device according to Claim 1, wherein said control valve also compensates said rate of flow for changes in ambient pressure.

3. The device according to either of Claims 1 or 2, wherein both of said displaceable members (10, 8; 210, 208) are diaphragms, and said pressure-control means comprises an electrolytic cell (26) having a pair of electrodes (28, 30; 228, 230) separated by an electrolyte (32; 232) capable of generating a gas applied to said second chamber (C₂) according to the electrical current passing through said electrolyte.

4. The device according to Claim 3, wherein the cross-sectional area of the second diaphragm (8; 208) is many times that of the outlet side of said control valve (18; 118; 218; 316).

5. The device according to Claim 4, wherein said compensating chamber includes electrical sensor means (222, 242; 222, 308) for sensing the opening of said control valve.

6. The device according to Claim 5, wherein said control valve comprises a valve member (218) and a valve seat (220) both carried by said second diaphragm (208); said valve seat having a yielding connection (222) with respect to said valve member (218) such that the valve member is normally closed with respect to the valve seat (220), but upon engagement of the second diaphragm (208) with a fixed part (240) of the housing, the valve member (218) moves with respect to the valve seat (220) to open the valve to said outlet; said electrical sensor means (222, 242; 222, 308) sensing the engagement of the second diaphragm (208) with respect to said fixed part (240) of the housing.

7. The device according to Claim 6, wherein said yielding connection comprises a stem (222) carried by said second diaphragm and engageable on one side thereof with said fixed part (240) of the housing, said valve member (218) also being carried by said second diaphragm (208); said stem (222) being displaceable with respect to said second diaphragm (208) and carrying the valve seat (220) on the opposite side of said diaphragm.

8. The device according to Claim 7, wherein said fixed part (240) of the housing includes a pressure-sensor (242) sensing the pressure applied by said stem against the fixed part of the housing.

9. The device according to any one of Claims 1-8, further including calibrating means for precalibrating the pressure required in said second chamber (C₂) for opening said control valve, said calibrating means comprising a rotatable member (240) rotatably carried by said housing and having an inclined surface engageable with said stem (222) carried by said second diaphragm (208).

10. The device according to any one of Claims 1-8, further including calibrating means for precalibrating the pressure required in said second chamber (C₂) for opening said control valve; said calibrating means comprising a leaf spring (300) fixed at its opposite ends to the housing, and a threaded pin (306) threaded through an opening in said housing and engageable with an intermediate portion of the leaf spring to move said intermediate portion towards or away from said opening in the housing.

## Patentansprüche

1. Flüssigkeitsabgabevorrichtung, die umfasst: ein äusseres Gehäuse (2; 102), das einen Auslass (14; 214) für die abzugebende Flüssigkeit hat; ein erstes verschiebbares Teil (10; 210) innerhalb des Gehäuses, das eine erste zusammenziehbare Kammer (C₁) auf einer Seite des ersten verschiebbaren Teils definiert, um einen Vorrat der abzugebenden Flüssigkeit zu enthalten, und eine zweite zusammenziehbare Kammer (C₂) auf der gegenüberliegenden Seite des ersten verschiebbaren Teils; und eine Drucksteuereinrichtung (26), um den Druck zu steuern, der in der zweiten zusammenziehbaren Kammer erzeugt wird, um die Verschiebung des ersten verschiebbaren Teils zu steuern und dadurch die Strömungsrate der Flüssigkeit über den Auslass; dadurch gekennzeichnet, dass das Gehäuse weiter umfasst: ein zweites verschiebbares Teil (8; 208), das eine dritte zusammenziehbare Kammer (C₃) zwischen dem Gehäuse und der ersten zusammenziehbaren Kammer in der Nähe des Auslasses definiert; und ein Steuerventil, das zwei Ventilteile (8a, 18; 8a, 118a; 218a, 220; 314, 316) hat, die senkrecht zueinander mit einem Zwischenraum angeordnet sind, um die Strömung zum Auslass zu steuern; wobei sich eines der Ventilteile (8a, 218a, 314) auf dem zweiten verschiebbaren Teil befindet, so dass Änderungen in der Umgebungstemperatur, die in der ersten und dritten Kammer erzeugt werden, im wesentlichen gleich grosse und entgegengesetzte Kräfte auf die gegenüberliegenden Seiten des verschiebbaren Teils erzeugen, wodurch das Steuerventil die Strömungsrate zum Auslass für Änderungen in der Umgebungstemperatur kompensiert.

2. Vorrichtung nach Anspruch 1, bei der das Steuerventil die Strömungsrate auch für Änderungen im Umgebungsdruck kompensiert

3. Vorrichtung nach irgend einem der Ansprüche 1 oder 2, bei der beide der verschiebbaren Teile (10, 8; 210, 208) Diaphragmen sind und die Drucksteuereinrichtung eine elektrolytische Zelle (26) umfasst, die ein Paar von Elektroden (28, 30; 228, 230) hat, die durch einen Elektrolyt (32; 232) getrennt sind, der fähig ist, gemäss des elektrischen Stroms, der durch den Elektrolyt hindurch geht, ein Gas zu erzeugen, das in die zweite Kammer (C₂) eingespeist wird.

4. Vorrichtung nach Anspruch 3, bei der die Querschnittfläche des zweiten Diaphragmas (8; 208) ein Vielfaches derjenigen der Auslassseite des Steuerventils (18; 118; 218; 316) beträgt.

5. Vorrichtung nach Anspruch 4, bei der die Kompensationskammer eine elektrische Messfühlereinrichtung (222, 242; 222, 308) zum Messen der Öffnung des Steuerventils umfasst.

6. Vorrichtung nach Anspruch 5, bei der das Steuerventil ein Ventilteil (218) und einen Ventilsitz (220) umfasst, die beide durch das zweite Diaphragma (208) getragen werden; wobei der Ventilsitz eine nachgebende Verbindung (222) bezüglich des Ventilteils (218) hat, so dass das Ventilteil normalerweise bezüglich des Ventilsitzes (220) geschlossen ist, wobei sich jedoch nach einer Berührung des zweiten Diaphragmas (208) mit einem festen Teil (240) des Gehäuses das Ventilteil (218) bezüglich des Ventilsitzes (220) bewegt, um das Ventil zum Auslass zu öffnen; wobei die elektrische Messfühlereinrichtung (222, 242; 222, 308) die Berührung des zweiten Diaphragmas (208) bezüglich des festen Teils (240) des Gehäuses misst.

7. Vorrichtung nach Anspruch 6, bei der die nachgebende Verbindung einen Stab (222) umfasst, der durch das zweite Diaphragma getragen wird und auf einer Seite von ihm den festen Teil (240) des Gehäuses berühren kann, wobei auch das Ventilteil (218) durch das zweite Diaphragma (208) getragen wird; wobei der Stab (222) bezüglich des zweiten Diaphragmas (208) verschoben werden kann und den Ventilsitz (220) auf der gegenüberliegenden Seite des Diaphragmas trägt.

8. Vorrichtung nach Anspruch 7, bei der das feste Teil (240) des Gehäuses einen Druckmessfühler (242) umfasst, der den Druck misst, der durch den Stab gegen den festen Teil des Gehäuses ausgeübt wird.

9. Vorrichtung nach irgend einem der Ansprüche 1 - 8, die weiter eine Eicheinrichtung umfasst, um den in der zweiten Kammer (C₂) zum Öffnen des Steuerventils erforderlichen Druck vorzueichen, wobei die Eicheinrichtung ein drehbares Teil (240) umfasst, das drehbar durch das Gehäuse getragen wird und eine geneigte Oberfläche hat, die durch den Stab (222), der durch das zweite Diaphragma (208) getragen wird, berührt werden kann.

10. Vorrichtung nach irgend einem der Ansprüche 1 - 8, die weiter eine Eicheinrichtung umfasst, um den in der zweiten Kammer (C₂) zum Öffnen des Steuerventils erforderlichen Druck vorzueichen; wobei die Eicheinrichtung eine Blattfeder (300) umfasst, die bei ihren gegenüberliegenden Enden am Gehäuse befestigt ist, und einen Gewindestift (306), der durch eine Öffnung im Gehäuse hindurch geschraubt ist und einen mittleren Teil der Blattfeder berühren kann, um den mittleren Teil gegen die Öffnung im Gehäuse hin oder von ihr weg zu bewegen.

## Revendications

1. Dispositif de distribution de liquide englobant: un logement externe (2; 202) comportant une sortie (14; 214) pour le liquide à distribuer; un premier élément (10; 210) apte à se déplacer dans le logement définissant une première chambre (C₁) apte à se contracter d'un côté du premier élément apte à se déplacer pour retenir une alimentation du liquide à distribuer, et une seconde chambre (C₂) apte à se contracter du côté opposé du premier élément apte à se déplacer; et un moyen de réglage de la pression (26) pour régler la pression générée dans ladite seconde chambre apte à se contracter dans le but de commander le déplacement du premier élément apte à se déplacer et par là, l'écoulement du liquide via ladite sortie; caractérisé en ce que ledit logement englobe en outre: un second élément (8; 208) apte à se déplacer définissant une troisième chambre (C₃) apte à se contracter entre ledit logement et ladite première chambre apte à se contracter en position adjacente à ladite sortie; et une soupape de réglage comportant deux éléments de soupape (8, 18; 8a, 118a; 218a, 220; 314, 316) normalement espacés l'un de l'autre pour régler l'écoulement en direction de ladite sortie; un desdits éléments de soupape (8a, 218a, 314) étant disposé sur ledit second élément apte à se déplacer de telle sorte que des changements intervenant dans la température ambiante générée dans les première et troisième chambres exercent des forces essentiellement égales et opposées sur les côtés opposés de l'élément apte à se déplacer de telle sorte que la soupape de réglage neutralise des changements de la température ambiante ayant une influence sur l'écoulement en direction de ladite sortie.

2. Dispositif selon la revendication 1, dans lequel ladite soupape de réglage neutralise également des changements de la pression ambiante auxquels est sensible ledit débit.

3. Dispositif selon, soit la revendication 1, soit la revendication 2, dans lequel les deux éléments précités (10, 8; 210, 208) aptes à se déplacer sont des diaphragmes et ledit moyen de réglage de la pression comprend une cellule électrolytique (26) possédant une paire d'électrodes (28, 30; 228, 230) séparées par un électrolyte (32; 232) apte à générer un gaz appliqué à ladite seconde chambre (C₂) en fonction du courant électrique traversant ledit électrolyte.

4. Dispositif selon la revendication 3, dans lequel l'aire de section du second diaphragme (8; 208) représente un multiple de celle côté sortie de ladite soupape de réglage (18; 118; 218; 316).

5. Dispositif selon la revendication 4, dans lequel ladite chambre de compensation englobe des moyens de détecteurs électriques (222, 242; 222, 308) pour détecter l'ouverture de ladite soupape de réglage.

6. Dispositif selon la revendication 5, dans lequel ladite soupape de réglage comprend un élément de soupape (218) et un siège de soupape (220), tous deux portés par ledit second diaphragme (208); ledit siège de soupape présentant une liaison (222) à déformation élastique avec ledit élément de soupape (218) de telle sorte que l'élément de soupape est normalement fermé par rapport au siège de soupape (220), mais lors de la mise en contact du second diaphragme (208) avec une partie fixe (240) du logement, l'élément de soupape (218) se déplace par rapport au siège de soupape (220) pour ouvrir la soupape en direction de ladite sortie; lesdits moyens de détecteurs électriques (222, 242; 222, 308) détectant la mise en contact du second diaphragme (208) avec ladite partie fixe (240) du logement.

7. Dispositif selon la revendication 6, dans lequel ladite liaison à déformation élastique comprend une tige (222) portée par ledit second diaphragme et apte à venir se mettre en contact à un de ses côtés avec ladite partie fixe (240) du logement, ledit élément de soupape (218) étant également porté par ledit second diaphragme (208); ladite tige (222) étant apte à se déplacer par rapport audit second diaphragme (208) et portant le siège de soupape (220) sur le côté opposé dudit diaphragme.

8. Dispositif selon la revendication 7, dans lequel ladite partie fixe (240) du logement englobe un détecteur de pression (242) détectant la pression exercée par ladite tige contre la partie fixe du logement.

9. Dispositif selon l'une quelconque des revendications 1 à 8, englobant en outre un moyen de calibrage pour soumettre à un précalibrage la pression requise dans ladite seconde chambre (C₂) pour ouvrir ladite soupape de réglage, ledit moyen de calibrage comprenant un élément rotatif (240) porté en rotation par ledit logement et possédant une surface inclinée apte à venir se mettre en contact avec ladite tige (222) portée par ledit second diaphragme (208).

10. Dispositif selon l'une quelconque des revendications 1 à 8, englobant en outre un moyen de calibrage pour soumettre à un précalibrage la pression requise dans ladite seconde chambre (C₂) pour ouvrir ladite soupape de réglage; ledit moyen de calibrage comprenant un ressort à lames (300) fixé à ses extrémités opposées au logement, et une broche filetée (306) vissée à travers une ouverture pratiquée dans ledit logement et apte à venir se mettre en contact avec une portion intermédiaire du ressort à lames pour déplacer ladite portion intermédiaire en direction ou à l'écart de ladite ouverture pratiquée dans le logement.
